(19) Europäisches Patentamt
European Patent Office
Office européen des brevets

(11) Veröffentlichungsnummer: **0 224 751**
**B1**

(12) # EUROPÄISCHE PATENTSCHRIFT

(45) Veröffentlichungstag der Patentschrift: **24.01.90**

(51) Int. Cl.⁴: **C07F 9/38,** C07F 9/40, A61K 31/66

(21) Anmeldenummer: **86115428.4**

(22) Anmeldetag: **07.11.86**

(54) **Neue Diphosphonsäurederivate, Verfahren zu deren Herstellung und diese Verbindungen enthaltende Arzneimittel.**

(30) Priorität: **13.11.85 DE 3540150**

(43) Veröffentlichungstag der Anmeldung:
**10.06.87 Patentblatt 87/24**

(45) Bekanntmachung des Hinweises auf die Patenterteilung:
**24.01.90 Patentblatt 90/4**

(84) Benannte Vertragsstaaten:
**AT BE CH DE ES FR GB GR IT LI LU NL SE**

(56) Entgegenhaltungen:
**EP-A- 0 001 584**
**FR-A- 2 187 292**
**GB-A- 2 118 042**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 116, D-6800 Mannheim 31(DE)**

(72) Erfinder: **Bosies, Elmar, Dr. rer. nat., Delpstrasse 11, D-6940 Weinheim(DE)**
Erfinder: **Gall, Rudi, Dr. phil., Ulmenstrasse 1, D-6945 Hirschberg 1(DE)**

**EP 0 224 751 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neue Diphosphonsäurederivate, Verfahren zu deren Herstellung sowie Arzneimittel, die diese Substanzen enthalten.

In der DE-A 1 813 659 sind Diphosphonsäurederivate beschrieben, von denen die 1-Hydroxy-ethan-1.1-diphosphonsäure als Mittel zur Behandlung von Morbus Paget Bedeutung erlangt hat. In der BE-A 896 453 sowie der EP-A 1 584 und EP-A 96 931 sind Aminoalkan-1.1-diphosphonsäuren als gute Calciumkomplexbildner beschrieben, die sich auch zur Behandlung der erhöhten Knochenresorption einsetzen lassen. Es wurde nun gefunden, daß Aminocycloalkan-diphosphonsäuren eine bisher nicht bekannte Gruppe von Calciumkomplexbildnern darstellen, die sich ebenfalls zur Behandlung der erhöhten Knochenresorption einsetzen lassen, und zwar vor allem sehr gut dort, wo der Knochenauf- und -abbau gestört ist, d.h. sie sind geeignet zur Behandlung von Erkrankungen des Skelettsystems, wie z.B. Osteoporose, Morbus Paget, Morbus Bechterew u.a.

Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Diphosphonate der allgemeinen Formel I

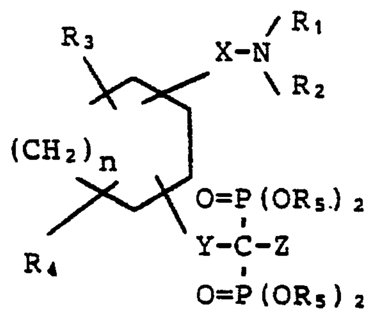

(I) ,

in der

$R_1$, $R_2$ jeweils unabhängig voneinander oder auch gemeinsam Wasserstoff, Acetyl oder $C_1$–$C_4$ Alkyl, das durch Phenyl, Pyridyl, Imidazolyl oder Thienyl substituiert sein kann,

$R_3$, $R_4$ jeweils unabhängig voneinander oder gemeinsam Wasserstoff, $C_1$–$C_4$ Alkyl oder zusammen $C_1$–$C_4$ Alkylen,

$R_5$ Wasserstoff oder $C_1$–$C_4$ Alkyl,

X einen Valenzstrich oder geradkettiges oder verzweigtes $C_1$–$C_4$ Alkylen,

Y einen Valenzstrich, geradkettiges oder verzweigtes, gegebenenfalls durch

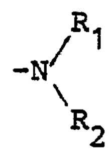

substituiertes $C_1$–$C_4$ Alkylen,

Z Wasserstoff, Hydroxy oder gegebenenfalls durch $C_1$–$C_4$ Alkylgruppen substituiertes Amino und

n = 1–3 bedeuten,

sowie deren pharmakologisch unbedenkliche Salze.

$C_1$–$C_4$-Alkyl bzw. Alkylen bei den Resten $R_1$ - $R_5$, X und Y, allein oder als Bestandteil anderer Substituenten, bedeutet vorzugsweise den Methyl-, Ethyl- und Isopropylrest bzw. die Methylen-, Ethylen- oder Propylidengruppe.

Unter einem Aryl-substituierten Alkylrest ist vorzugsweise der Benzyl- oder Pyridylmethylrest zu verstehen.

$R_5$ bedeutet vorzugsweise Wasserstoff.

n = 1-3 stellen die Cyclopentyl-, Cyclohexyl- oder Cycloheptylreste dar, vorzugsweise einen Cyclohexylrest.

Bei durch $R_3$ - $R_4$ überbrückten Ringen ist der Norbornyl- und der Bicyclo[2.2.2]octylrest bevorzugt.

Die Cycloalkane können als Stereoisomeren-Gemische oder als reine cis- bzw. trans-Isomere vorliegen.

In X, Y oder den Ringen vorkommende asymmetrische Kohlenstoffatome können die R-, S- oder R, S-Konfiguration besitzen.

Verbindungen der allgemeinen Formel I werden nach an sich bekannten Verfahren hergestellt.

I. Für den Fall, daß Z in der allgemeinen Formel I OH bedeutet, stellt man die Substanzen vorzugsweise dadurch her, daß man
a) eine Carbonsäure der allgemeinen Formel II

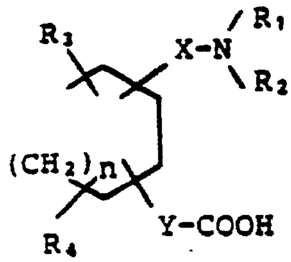

(II),

in der $R_1$ - $R_4$, X, Y und n die oben angegebenen Bedeutungen haben, mit einem Gemisch aus phosphoriger Säure bzw. Phosporsäure und einem Phosphorhalogenid umsetzt und anschließend zur freien Diphosphonsäure verseift,
oder
b) ein Carbonsäurechlorid der allgemeinen Formel III

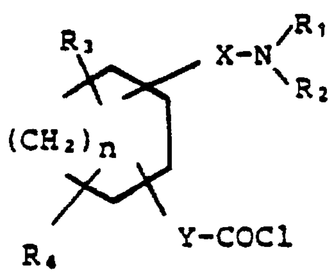

(III),

in der $R_1$, $R_2$, $R_3$, $R_4$, X, Y und n die oben angegebenen Bedeutungen haben, und falls

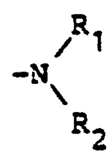

eine primäre oder sekundäre Aminogruppe darstellt, diese Gruppe geschützt ist, wobei $R_1$ und $R_2$ zusammen auch den Phthaloylrest bedeuten können, mit einem Trialkylphosphit der allgemeinen Formel IV

$P(OR')_3$ (IV),

in der R' niederes Alkyl bedeutet, zu einem Acylphosphonat der allgemeinen Formel V

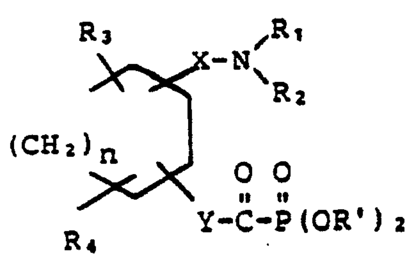

(V),

in der $R_1$ - $R_4$, X, Y, n und R' die oben angegebenen Bedeutungen haben,
umsetzt, anschließend mit einem Dialkylphosphit der allgemeinen Formel VI

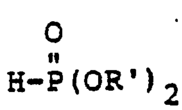

(VI),

in der R' die oben angegebene Bedeutung hat, zu einem Diphosphonat der allgemeinen Formel VII

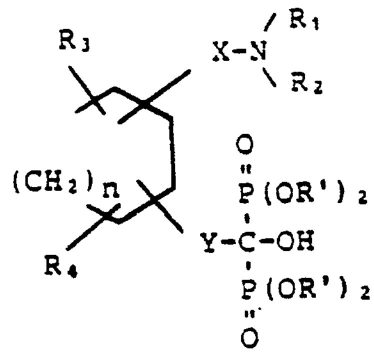

(VII),

in der $R_1$ - $R_4$, X, Y, n und R' die oben angegebenen Bedeutungen haben, reagieren läßt, gegebenenfalls vorhandene Schutzgruppen abspaltet und gegebenenfalls die entstandenen Tetraester zu Diestern oder Säuren der allgemeinen Formel I verseift,
oder
c) eine Verbindung der allgemeinen Formel I'

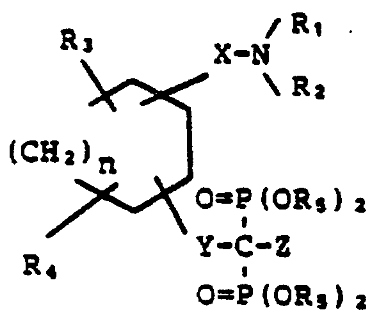

(I'),

in der $R_3$, $R_4$, $R_5$, n, X und Y die oben angegebenen Bedeutungen haben, $R_1$ und $R_2$ jeweils niederes Acyl oder Alkyl, das durch Aryl substituiert sein kann oder zusammen einen Phthaloylrest bedeuten und Z = $NH_2$ darstellt,
diazotiert
oder
II. für den Fall, daß Z in der allgemeinen Formel I gegebenenfalls durch Alkylgruppen substituiertes Amino bedeutet,
ein Carbonsäurederivat der allgemeinen Formel VIII

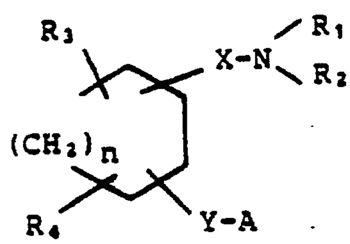

(VIII),

in der $R_1$, - $R_4$, X, Y und n die oben angegebenen Bedeutungen haben und A eine Nitril-, Iminoether- oder eine gegebenenfalls am Stickstoff durch niederes Alkyl substituierte Carboxamidogruppe darstellt,
mit einer Phosphorverbindung der allgemeinen Formel IX

$$PT_3 \qquad \text{(IX)},$$

in der T = Halogen, OH oder OR' bedeutet, wobei R' die oben angegebene Bedeutung hat, umsetzt und gegebenenfalls anschließend verseift
oder

III. für den Fall, daß Z in der allgemeinen Formel I Wasserstoff bedeutet, eine Verbindung der allgemeinen Formel X

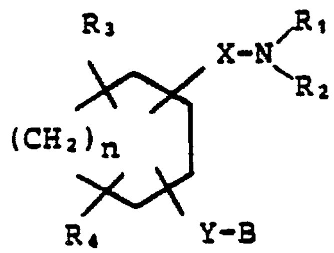

(X),

in der $R_1$ - $R_4$, X, Y und n die oben angegebenen Bedeutungen haben und falls

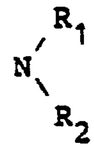

eine primäre oder sekundäre Aminogruppe darstellt, diese Gruppe geschützt ist, wobei $R_1$ und $R_2$ zusammen auch den Phthaloylrest bedeuten können, und B einen reaktiven Rest wie z.B. Halogen oder ein Sulfonat darstellt, mit einer Verbindung der allgemeinen Formel XI

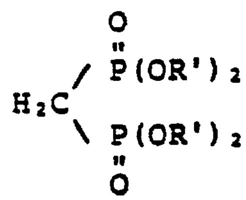

(XI),

in der R' die oben angegebene Bedeutung hat, zu einem Diphosphonat der allgemeinen Formel XII

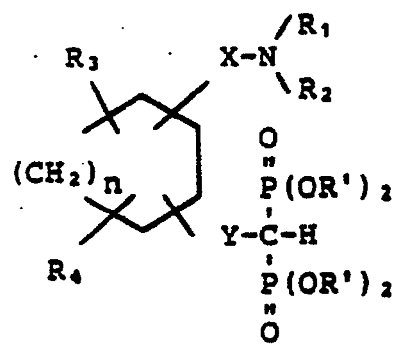

(XII),

in der $R_1$ - $R_4$, X, Y, n und R' die oben angegebenen Bedeutungen haben, umsetzt, gegebenenfalls vorhandene Schutzgruppen abspaltet und gegebenenfalls die entstandenen Tetraester zu Diestern oder Säuren der allgemeinen Formel I verseift und gewünschtenfalls vorhandene N-Acyl-Substituenten abspaltet und anschließend gewünschtenfalls freie Aminogruppen alkyliert oder acyliert und die erhaltenen Verbindungen der Formel I in pharmakologisch verträgliche Salze überführt.

Bei Verfahren III) setzt man den Methylendiphosphonsaeureester der allgemeinen Formel XI in Form seines Natrium- oder Kaliumsalzes ein. Hierzu wird er mit Natrium, Kalium oder dem entsprechenden Hydrid in einem inerten Loesungsmittel wie z.B. Benzol, Toluol oder Dimethylformamid bei einer Temperatur von 0 bis 40°C, vorzugsweise bei 25°C umgesetzt. Das Alkalisalz wird ohne Isolierung mit dem entsprechenden Halogenid bzw. Sulfonat zur Reaktion gebracht. Die Temperatur liegt hierbei bei 20 - 110°C.

Bei Verfahren II) setzt man die Nitrile der allgemeinen Formel VIII mit phosphoriger Saeure bei Temperaturen von110 - 180°C um. Die Reaktion kann ohne oder in Gegenwart von aprotischen Loesungsmitteln

wie z.B. Diglykoldimethylether oder Diglykoldiethylether durchgefuehrt werden. Man kann die Nitrile jedoch auch mit einem Phosphortrihalogenid, z.B. Phosphortribromid oder Phosphortrichlorid in einem inerten Loesungsmittel wie z.B. Dioxan oder Tetrahydrofuran gegebenenfalls unter Zusatz von Wasser bei Temperaturen von 20 - 80°C zur Reaktion bringen. Iminoether der allgemeinen Formel VIII laeßt man mit Dialkylphosphiten vorzugsweise in Gegenwart aequimolarer Mengen Natrium in inerten Loesungsmitteln wie Diethylether, Dioxan oder auch Benzol reagieren, wobei die Umsetzungen in der Regel bei der Rueckflußtemperatur des entsprechenden Loesungsmittels stattfindet. Saeureamide der allgemeinen Formel VIII kann man in inerten Loesungsmitteln wie z.B. halogenierten Kohlenwasserstoffen oder Ethern wie z.B. Diethylether mit einem Gemisch aus Phosphorpentahalogenid/phosphoriger Saeure oder auch Oxalylchlorid/Trialkylphosphit umsetzen.

Die bei Verfahren I a) eingesetzten Carbonsaeuren der allgemeinen Formel II werden mit 1-2, vorzugsweise 1.5 Mol phosphoriger Säure bzw. Phosphorsäure und 1-2, vorzugasweise 1.5 Mol Phosphortrihalogenid bei Temperaturen von 80 - 130°C, vorzugsweise 100 - 110°C umgesetzt. Man kann die Reaktion auch in Gegenwart von Verdünnungsmitteln wie Halogenkohlenwasserstoffen, insbesondere Chlorbenzol, Tetrachlorethan oder auch Dioxan durchführen. Die anschließende Hydrolyse erfolgt durch Kochen mit Wasser, zweckmäßigerweise jedoch mit halbkonzentrierter Salz- oder Bromwasserstoffsäure.

Bei Verfahren I b) läßt man das Säurechlorid der allgemeinen Formel III mit dem Trialkylphosphit der allgemeinen Formel IV bei Temperaturen zwischen 0 und 60° C, vorzugsweise bei 20 - 40° C zur Reaktion kommen. Mann kann ohne Lösungsmittel oder auch in Gegenwart von inerten Loesungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel V kann isoliert oder direkt weiter umgesetzt werden. Die anschließende Reaktion fuehrt man in Gegenwart einer schwachen Base, vorzugsweise einem sec. Amin wie z.B. Dibutylamin bei einer Temperatur von 0 - 60°C, vorzugsweise bei 10 - 30°C durch.

Bei Verfahren I c) läßt man auf eine 1-Amino-1.1-diphosphonsäure der allgemeinen Formel I' in wäßriger Lösung Natriumnitrit im Überschuß, vorzugsweise 4 Mol, bei Temperaturen zwischen 0° und 60°C, vorzugsweise bei 20°-40°C, einwirken.

Die in den oben erlaeuterten Verfahren verwendeten Schutzgruppen sind aus der Peptidchemie bekannt und z.B. in Houben/Weyl Band 15/1 ausfuehrlich beschrieben. Bevorzugt werden als Schutzgruppen Aralkyloxycarbonylgruppen, insbesondere Benzyloxycarbonyl, oder Alkoxycarbonylgruppen, bevorzugt tert.-Butyloxycarbonyl. Es kann jedoch auch eine Formyl-, Trityl-, Trifluoracetyl- oder die Trichlorethoxycarbonylgruppe verwendet werden.

Die Abspaltung der Schutzgruppen nach erfolgter Umsetzung kann in ueblicher Weise durchgefuehrt werden. Benzyloxycarbonyl- und Tritylgruppen lassen sich durch katalytische Hydrierung in Gegenwart von Edelmetallkatalysatoren wie z.B. Palladium auf Kohle, tert.-Butoxycarbonylgruppen durch Einwirkung von starken Saeuren wie z.B. Salzsaeure oder Trifluoressigsaeure abspalten. Trichlorethoxycarbonylgruppen sind reduktiv, z.B. durch Einwirkung von Zink in Eisessig spaltbar. Die Phthaloylgruppe laeßt sich in saurem Medium oder auch durch Hydrazinolyse entfernen.

Die bei Verfahren Ib) II und III gegebenenfalls anfallenden Tetraalkylester koennen zu Diestern oder den freien Tetrasaeuren verseift werden. Die Verseifung zu Diestern geschieht in der Regel dadurch, daß man den Tetraalkylester mit einem Alkalihalogenid, vorzugsweise Natriumjodid in einem geeigneten Loesungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disaeure umgewandelt werden kann. Die Verseifung zu freien Diphosphonsaeuren geschieht in der Regel durch Kochen mit Salz- oder Bromwasserstoffsaeure. Man kann jedoch auch eine Spaltung mit Trimethylsilylhalogenid, vorzugsweise dem Bromid oder Jodid vornehmen. Die freien Diphosphonsaeuren koennen umgekehrt durch Kochen mit Orthoameisensaeurealkylestern wieder in die Tetraalkylester ueberfuehrt werden. Die freien Diphosphonsaeuren der allgemeinen Formel I koennen als freie Saeuren oder in Form ihrer Mono- oder Dialkalisalze isoliert werden. Die Alkalisalze lassen sich in der Regel durch Umfaellen aus Wasser/Methanol oder Wasser/Aceton gut reinigen.

Die Verbindungen der allgemeinen Formel I koennen gegebenenfalls nachtraeglich ineinander ueberfuehrt werden. Sie koennen z.B. alkyliert oder acyliert werden. Durch hydrogenolytische Abspaltung einer N-Benzylgruppe lassen sich z.B. die entsprechenden unsubstituierten Verbindungen der allgemeinen Formel I darstellen.

Als pharmakologisch vertraegliche Salze werden vor allem Alkali- oder Ammoniumsalze verwendet, die man in ueblicher Weise z.B. durch Neutralisieren der Verbindungen mit anorganischen oder organischen Basen wie z.B. Natrium- oder Kaliumhydrogencarbonat, Natronlauge, Kalilauge, waessrigem Ammoniak oder Aminen wie z.B. Trimethyl- oder Triethylamin herstellt.

Die erfindungsgemaeßen neuen Substanzen der Formel I und ihre Salze koennen in fluessiger oder fester Form enteral oder parenteral appliziert werden. Hierbei kommen alle ueblichen Applikationsformen in Frage, beispielsweise Tabletten, Kapseln, Dragees, Sirupe, Loesungen, Suspensionen etc. Als Injektionsmedium kommt vorzugweise Wasser zur Anwendung, welches die bei Injektionsloesungen ueblichen Zusaetze wie Stabilisierungsmittel, Loesungvermittler und Puffer enthaelt. Derartige Zusaetze sind z.B. Tartrat- und Citrat-Puffer, Ethanol, Komplexbildner (wie Ethylendiamintetraessigsaeure und

deren nichttoxische Salze), hochmolekulare Polymere (wie fluessiges Polyethylenoxid) zur Viskositaetsregelung. Fluessige Traegerstoffe fuer Injektionsloesungen muessen steril sein und werden vorzugsweise in Ampullen abgefuellt. Feste Traegerstoffe sind z.B. Staerke, Lactose, Mannit, Methylcellulose, Talkum, hochdisperse Kieselsaeuren, hoehermolekulare Fettsaeuren (wie Stearinsaeure), Gelatine, Agar-Agar, Calciumphosphat, Magnesiumstearat, tierische und pflanzliche Fette, feste hochmolekulare Polymere (wie Polyethylenglykole); fuer orale Applikation geeignete Zubereitungen koennen gewuenschtenfalls Geschmacks- und Sueßstoffe enthalten.

Die Dosierung kann von verschiedenen Faktoren, wie Applikationsweise, Spezies, Alter und/oder individuellem Zustand abhaengen. Die taeglich zu verabreichenden Dosen liegen bei etwa 1 - 1000 mg/Mensch, vorzugsweise bei 10 - 200 mg/Mensch und koennen auf einmal oder mehrere Male verteilt eingenommen werden.

Bevorzugt im Sinne der vorliegenden Erfindung sind außer den in den Beispielen genannten Verbindungen und den durch Kombination aller in den Anspruechen genannten Bedeutungen ableitbaren Verbindungen die folgenden Diphosphonate sowie deren Methyl- oder Ethylester:

3-(2-Aminocycloheptyl)-1-hydroxypropan-1,1-diphosphonsaeure
3-(2-N-Benzylaminocyclohexyl)-1-hydroxypropan-1,1-diphosphonsaeure
2-(2-Aminocyclohexyl)-1-hydroxyethan-1,1-diphosphonsaeure

1-[3-(2-Aminoethyl)cyclohexyl]-1-hydroxymethan-1,1-diphosphonsaeure
1-[2-(2-Aminoethyl)cyclohexyl]-1-hydroxymethan-1,1-diphosphonsaeure
1-(2-Aminomethylcyclohexyl)-1-hydroxymethan-1,1-diphosphonsaeure
3-(1-Aminocyclopentyl)-1-hydroxypropan-1,1-diphosphonsaeure
3-(1-Aminocycloheptyl)-1-hydroxypropan-1,1-diphosphonsaeure

cis-3-(2-Benzylaminocyclohexyl)-1-hydroxypropan-1,1-diphosphonsaeure
trans-3-(2-Benzylaminocyclohexyl)-1-hydroxypropan-1,1-diphosphonsaeure
3-(3-Amino-2-norbornyl)-1-hydroxypropan-1,1-diphosphonsaeure
2-(3-Amino-2-norbornyl)-1-hydroxyethan-1,1-diphosphonsaeure
3-(3-Amino-2-bicyclo[2,2,2]octyl)-1-hydroxypropan-1,1-diphosphonsaeure
2-(3-Amino-2-bicyclo[2,2,2]octyl)-1-hydroxyethan-1,1-diphosphonsaeure
3-Amino-3-(2-aminocyclohexyl)-1-hydroxypropan-1,1-diphosphonsaeure
2-Amino-3-(2-aminocyclohexyl)-1-hydroxypropan-1,1-diphosphonsaeure
1-Hydroxy-3-(2-methylaminocyclohexyl)propan-1,1-diphosphonsaeure
1-Amino-3-(2-aminocyclohexyl)propan-1,1-diphosphonsaeure
2-(2-Aminocyclohexyl)ethan-1,1-diphosphonsaeure
1-Hydroxy-3-[2-(2-pyridiylmethylamino)cyclohexyl]propan-1,1-diphosphonsaeure
3-(2-Aminocyclohexyl)propan-1,1-diphosphonsaeure
3-(1-N,N-Dimethylamino-cyclohexyl)-1-hydroxypropran-1,1-diphosphonsäure

Die nachfolgenden Beispiele zeigen einige der Verfahrensvarianten, die zur Synthese der erfindungsgemaeßen Verbindungen verwendet werden koennen. Sie sollen jedoch keine Einschraenkung des Erfindungsgegenstandes darstellen. Die Struktur dieser Verbindungen ist durch H- und P-NMR-Spektroskopie gesichert, die Reinheit mittels P-NMR-Spektroskopie, Duennschichtelektrophorese (Cellulose, Oxalat-Puffer von pH = 4.0) und mittels C,H,N,P,Na-Analyse bestimmt. Zur Charakterisierung der einzelnen Substanzen werden die $M_{rel}$-Werte (= relative Mobilitaet) bezogen auf Pyrophosphat ($M_{rel}$ = 1.0) angegeben.

Beispiel 1

2-(4-Aminocyclohexyl)-1-hydroxyethan-1,1-diphosphonsaeure

β-Nitrophenylessigsaeure wird mit Raney-Nickel in Ethanol hydriert und gibt mit 72 % Ausbeute p-Aminophenylessigsaeure, Fp. 185-189°C. Diese wird in salzsaurer Loesung mit Platinkatalysator zu p-Aminocyclohexylessigsaeure hydriert, Ausbeute: 23 % Hydrochlorid, Fp. 164-168°C.

1.7 g p-Aminocyclohexylessigsaeure-Hydrochlorid werden in 10 ml Chlorbenzol suspendiert, 1 g phosphorige Saeure zugegeben, auf 100°C gebracht und unter Ruehren 2,3 ml Phosphortrichlorid zugetropft. Nach 12 Stunden Ruehren bei 100°C kuehlt man ab, dekantiert das Loesungsmittel ab, versetzt den Rueckstand mit 15 ml 6 N Salzsaeure und ruehrt 6 Stunden bei 100°C. Man kuehlt die Suspension ab, versetzt mit Aceton, engt die Loesung ein und bringt den Rueckstand mit Methanol zur Kristallisation. Man erhaelt so 1.2 g = 45 % der Diphosphonsaeure als Halbhydrat, Fp. 223-228°C Zers. ($M_{rel}$: 0.22).

1.57 g dieser Diphosphonsaeure werden in 20.7 ml 1 N Natronlauge geloest, mit 1 ml Acetanhydrid versetzt und 2 Tage bei Raumtemperatur stehen gelassen. Dann gibt man die Reaktionsloesung auf eine Amberlite®-Saeule (IR 120-$H^+$Form) und eluiert mit Wasser, das eingedampft wird. Der oelige Rueckstand wird mit Methanol zur Kristallisation gebracht, Fp. 208-213°C Zers. Man erhaelt so 0.6 g = 33 % 2-(4-Acetylaminocyclohexyl)-1-hydroxyethan-1,1-diphosphonsaeure als Monohydrat. ($M_{rel}$: 0.61).

Beispiel 2

3-(4-Aminocyclohexyl)-1-hydroxypropan-1,1-diphosphonsäure

p-Aminocyclohexylpropionsaeure wird aus 4-Nitrozimtsaeure durch Hydrierung erhalten (26 % Ausbeute, Fp. 195-201°C) und wie in Beispiel 1 beschrieben mit Phosphortrichlorid/phosphoriger Saeure umgesetzt. Mit 45 % Ausbeute erhaelt man die gewuenschte Diphosphonsaeure, Fp. 255-258°C, die mit 1.5 Mol Wasser kristallisiert. $M_{rel}$: 0.22.

Beispiel 3

1-(4-Aminocyclohexyl)-1-hydroxymethan-1,1-diphosphonsaeure

p-Aminobenzoesaeure wird mit Platinkatalysator hydriert. Man erhaelt mit 67 % Ausbeute 4-Aminocyclohexylcarbonsaeure, Fp. 256-258°C. Die Umsetzung mit Phosphortrichlorid/phosphoriger Saeure erfolgt wie in Beispiel 1 beschrieben und liefert die gewuenschte Verbindung mit 32 % Ausbeute, Fp. 235-238°C Zers., $M_{rel}$: 0.27.

Beispiel 4

trans-1-(4-Aminomethylcyclohexyl)-1-hydroxymethan-1,1-diphosphonsaeure-mono-Natriumsalz

trans-4-Aminomethylcyclohexancarbonsaeure (Fa. Aldrich) wird wie in Beispiel 1 beschrieben mit Phosphortrichlorid/phosphoriger Saeure umgesetzt, die mit 33 % Ausbeute erhaltene Diphosphonsaeure (Fp. 220-225°C) wird in Wasser suspendiert, mit 1 N Natronlauge auf pH 5 gebracht, mit Aceton ausgefaellt und mit Methanol zur Kristallisation gebracht (Fp. 275-294°C) Ausbeute: 90 % Mono-Natriumsalz, $M_{rel}$: 0.26.

Durch Ruehren des Tetra-Natriumsalzes dieser Diphosphonsaeure in Wasser mit Acetanhydrid bei Raumtemperatur und anschließende Amberlite-Behandlung wie in Beispiel 1 beschrieben, erhaelt man mit 44 % Ausbeute die 1-(4-Acetylaminomethylcyclohexyl)-1-hydroxymethan-1,1-diphosphonsaeure (Fp. 164°C sintern, 190°C Zers. $M_{rel}$: 0.59) als Halbhydrat.

Beispiel 5

2-(4-N,N-Dimethylaminocyclohexyl)-1-hydroxyethan-1,1-diphosphonsaeure-di-Natriumsalz

Die in Beispiel 1 beschriebene p-Aminocyclohexylessigsaeure wird in waessriger Loesung mit Formalinloesung versetzt und mit Palladiumkohle zu 4-N,N- Dimethylaminocyclohexylessigsaeure hydriert (Ausbeute: 24 %, Fp. bei 163°C sintern, bei 179°C geschmolzen).

Die Umsetzung zur Diphosphonsaeure erfolgt nach den in Beispiel 1 gemachten Angaben (Ausbeute: 83 %, Fp. 60-65°C Zers.). Mit der berechneten Menge 1 N Natronlauge wird das Di-Natriumsalz als Monohydrat in 43 % Ausbeute erhalten (Fp. bei 350°C aufblaehen, $M_{rel}$: 0.24).

Beispiel 6

2-(4-Aminomethylcyclohexyl)-1-hydroxyethan-1,1-diphosphonsaeure

Die in Beispiel 1 beschriebene p-Aminophenylessigsaeure wird nach den Angaben in J.Chem.Soc. 1941,745 durch Diazotieren in p-Cyanphenylessigsaeure ueberfuehrt (Ausbeute: 47 %, Fp. 138-141°C) und mit Platinkatalysator in salzsaurer Loesung zur 4-Aminomethylcyclohexylessigsaeure hydriert (Ausbeute: 63 %, Fp. 120-123°C Zers.).

Mit 35 % Ausbeute wird daraus in analoger Weise wie in Beispiel 1 beschrieben die Diphosphonsaeure als Halbhydrat erhalten, Fp. 208-214°C Zers., $M_{rel}$: 0.24).

Beispiel 7

2-(3-Aminocyclohexyl)-1-hydroxyethan-1,1-diphosphonsaeure

3-Aminocyclohexylessigsaeure wird in analoger Weise wie in Beispiel 1 beschrieben zum Diphosphonsaeure-Dihydrat umgesetzt (Ausbeute: 11 %, Fp. 223-228°C Zers., $M_{rel}$: 0.26).

Die als Ausgangsmaterial eingesetzte 3-Aminocyclohexylessigsaeure wird wie folgt erhalten: 3-Nitrobenzaldehyd wird nach den Angaben in JACS 71,123 mit Natriumborhydrid in Methanol zum 3-Nitrobenzylalkohol reduziert, Ausbeute: 97 %, schmierige Kristalle. Dieser wird nach JACS 52,1643 mittels Thionylchlorid zum 3-Nitrobenzylchlorid umgesetzt (Ausbeute: 42 %, Fp. 40-42°C aus Ligroin).

Die Umsetzung mit Kaliumcyamid wird nach Chem.&Ind. 1935,105 durchgefuehrt und gibt mit 58 % Ausbeute 3-Nitrophenylessigsaeure, Fp. 101-107°C. Daraus erhaelt man durch Hydrierung mit Platinkatalysator die gewuenschte 3-Aminocyclohexylessigsaeure (Ausbeute: 28 %, Fp. 188-200°C Zers.).

Beispiel 8

2-(3-Aminomethylcyclohexyl)-1-hydroxyethan-1,1-diphosphonsaeure

Die in Beispiel 7 beschriebene 3-Nitrophenylessigsaeure wird in Methanol mit Palladium-Kohle hydriert und gibt mit 99 % Ausbeute 3-Aminophenylessigsaeure (Fp. 116-121°C). In analoger Weise wie fuer die p-Verbindung beschrieben (s. Beispiel 6) erhaelt man daraus durch Diazotieren die 3-Cyanphenylessigsaeure (Ausbeute: 45 %, Fp. 93-98°C Zers.), durch Hydrieren mit Platinkatalysator in verd. Salzsaeure die 3-Aminomethylcyclohexylessigsaeure (Ausbeute: 66 %, Fp. 127-130°C). Die Diphosphonsaeure wird mittels Phosphortrichlorid/phosphoriger Saeure entsprechend Beispiel 1 als Monohydrat gewonnen. (Ausbeute: 28 %, Fp. 216-221°C Zers., $M_{rel}$: 0.27).

Beispiel 9

3-(3-Aminocyclohexyl)-1-hydroxypropan-1,1-diphosphonsaeure

m-Zimtsaeure wird in salzsaurer Loesung mit Palladium-Kohle hydriert und die erhaltene β-(3-Aminophenyl)propionsaeure mit Platinkatalysator weiter hydriert. Ausbeute: 56 % β-(3-Aminocyclohexyl)propionsaeure, Fp. 175-185°C Zers.

Die nach Beispiel 1 erhaltene Diphosphonsaeure wird mit 66 % Ausbeute als Halbhydrat erhalten, Fp. 221-223°C Zers., $M_{rel}$: 0.23).

Beispiel 10

3-(2-Acetylaminocyclohexyl)-1-hydroxypropan-1,1-diphosphonsaeure

o-Nitrozimtsaeure wird in 1 N-Natronlauge mit Raney-Nickel hydriert. Zum Filtrat wird anschließend Acetanhydrid gegeben, ueber Nacht stehen gelassen, die entstandene Suspension abgesaugt, gewaschen und getrocknet. Ausbeute: 57 % o-Acetylaminohydrozimtsaeure, Fp. 139-143°C.

Nach Hydrierung in salzsaurer Loesung mit Platinkatalysator erhaelt man 3-(2-Acetylaminocyclohexyl)propionsaeure als cis-trans-Gemisch mit einem trans-Anteil von 11 % (Ausbeute: 50 %, Fp. 116-121°C).

Die Diphosphonsaeureherstellung erfolgte nach den Angaben von Beispiel 1, jedoch wurde nach der salzsauren Hydrolyse das Produkt, das teilweise entacetyliert war, durch Behandeln mit Acetanhydrid zur einheitlichen 3-(2-Acetylaminocyclohexyl)-1-hydroxypropan-1,1-diphosphonsaeure umgesetzt (Ausbeute: 38 % Monohydrat, Fp. ab 83°C aufschaeumen, $M_{rel}$: 0.56).

Beispiel 11

2-(2-Aminomethylcyclohexyl)-1-hydroxyethan-1,1-diphosphonsaeure

(2-Aminomethylcyclohexyl)essigsaeure wird analog Beispiel 1 mit Phosphortrichlorid/phosphoriger Saeure zur Diphosphonsaeure, die als Halbhydrat erhalten wird, umgesetzt. Ausbeute: 4 % nach Reinigen an der Amberlite-Saeule (IR 120 $H^+$ Form). Fp. 196°C aufschaeumen, $M_{rel}$: 0.32.

Das Ausgangsmaterial wird wie folgt erhalten: 2-Indanonoxim wird nach J.Org.Chem. 9, 386 mit 81 % Ausbeute erhalten, Fp. 145-148°C. Die Umlagerung zum Lactam der 2-Aminomethylphenylessigsaeure wird nach J.Chem.Soc. 65,490 mittels Phosphorpentachlorid erreicht. (Ausbeute 69 %). Die Ringoeffnung zur 2-Aminomethylphenylessigsaeure erfolgt durch 2-stuendiges Kochen in konz. Salzsaeure mit 22 % Ausbeute, Fp. 151-155°C (Hydrochlorid). Schließlich wird durch Hydrieren mit Platinkatalysator in salzsaurer Loesung mit 98 % Ausbeute das Hydrochlorid der (2-Aminomethylcyclohexyl)essigsaeure erhalten, schaumiges Produkt.

Beispiel 12

1-(3-Aminomethylcyclohexyl)-1-hydroxymethan-1,1-diphosphonsaeure

m-Cyanbenzoesaeure wird nach J.prakt.Chem. 125,43 aus m-Aminobenzoesaeure durch Diazotieren mit 52 % Ausbeute hergestellt, Fp. 210-212°C. Durch Hydrieren in essigsaurer Loesung mit Palladium-Kohle erhaelt man m-Aminomethylbenzoesaeure und Hydrieren mit Platinkatalysator in salzsaurer Loesung gibt 3-Aminomethylcyclohexancarbonsaeure (Ausbeute: quantitativ, schmierige Kristalle).

Die Diphosphonsaeureherstellung erfolgt analog Beispiel 1. Das Monohydrat wird mit 33 % Ausbeute erhalten, Fp. 208-212°C, $M_{rel}$: 0.32.

Beispiel 13

cis-3-(2-Aminocyclohexyl)-1-hydroxypropan-1,1-diphosphonsäure

Aus dem im Beispiel 10 beschriebenen cis-trans-Gemisch der 3-(2-Acetylaminocyclohexyl)propionsäure erhält man nach Umkristallisieren aus Wasser die reine cis-Verbindung (Ausbeute: 32 %, Fp. 131-133°C), deren Umsetzung mit Phosphortrichlorid/phosphoriger Säure analog Beispiel 1 erfolgt. Der Verlauf der Abspaltung der Acetylgruppe durch Erhitzen in Salzsäure wird mittels Dünnschichtelektrophorese verfolgt.

Beispiel 14

1-[4-(2-Aminoethyl)cyclohexyl]-1-hydroxymethan-1,1-diphosphonsaeure

In analoger Weise wie in Beispiel 1 beschrieben erhaelt man aus der 4-(2-Aminoethyl)-cyclohexancarbonsäure mit 32 % Ausbeute die gewuenschte Verbindung (Fp. 226-230°C, $M_{rel}$: 0.21). Das Ausgangsmaterial wird wie folgt erhalten: p-Brommethylbenzoesaeure, Fp. 215-218°C, aus p-Toluylsaeure mit N-Bromsuccinimid nach J.Org. Chem. 18,708 mit 66 % Ausbeute erhalten, wird nach Veresterung mit Methanol und Thionylchlorid (84 % Ausbeute) mit Kaliumcyanid nach J.Org.Chem. 17,1037 zum p-Cyanmethylbenzoesaeuremethylester umgesetzt (Ausbeute: 59 %, Fp. 56-59°C). Die Hydrierung mit Palladium-Kohle liefert mit 64 % Ausbeute den 4-(2-Aminoethyl)-benzoesaeuremethylester, Fp. 209-216°C Zers.
Die weitere Hydrierung mit Platinkatalysator und anschließende Verseifung mit 2 N Natronlauge bei Raumtemperatur gibt die 4-(2-Aminoethyl)cyclohexancarbonsaeure, Fp. 218-224°C Zers., Ausbeute: 65%.

Beispiel 15

cis-3-(2-Aminocyclohexyl)-1-hydroxypropan-1,1-diphosphonsäure, die mit der nach Beispiel 13 hergestellten Verbindung identisch ist, erhält man auch durch Umsetzung des Hydrochlorids der cis-3-(2-Aminocyclohexyl)propionsäure mit Phosphortrichlorid/phosphoriger Säure analog Beispiel 1 und Reinigung des Hydrolyseprodukts durch Ionenaustauscherchromatographie. Ausbeute: 10 %, Fp. 204°C sintern, ab 208°C aufschäumen.
Das Ausgangsmaterial wird wie folgt erhalten:
o-Nitrozimtsäure wird nach Bull.Soc.chim. France l964,2617 hydriert und das mit 93 % Ausbeute gebildete 1,2,3,4-Tetrahydrochinolin-2-on, Fp. 150-155 °C mit Platinkatalysator in salzsaurer Lösung zum Dekahydrochinolin-2-on hydriert (Ausbeute: 79 %). Aus dem cis-trans-Gemisch (ca. 10 % trans) erhält man durch zweimaliges Umkristallisieren aus Wasser die reine cis-Verbindung (Ausbeute: 23 %, Fp. 158-163°C). Durch Kochen mit Salzsäure wird der Lactamring geöffnet (Ausbeute: 59 %, Fp. 161-163°C).

Beispiel 16

3-(1-Aminocyclohexyl)-1-hydroxypropan-1,1-diphosphonsaeure

19,1 g 1-Aza-spiro[4,5]decan-2-on (nach J. Org. Chem. 22, 832 hergestellt) werden mit 190 ml conz. Salzsaeure 90 Stunden gekocht. Nach Abkuehlen wird filtriert, eingeengt und der Rueckstand mit Methanol/Diethylether umgefaellt. Man erhaelt 17,5 g = 68 % des Hydrochlorids der 3-(1-Aminocyclohexyl)propionsaeure, Fp. 183° C sintern, 188 - 192°C schmelzen unter Zersetzung. 7 g davon werden mit 5,5 g phosphoriger Saeure vermischt und nach Schmelzen bei 80° c mit 5,9 ml Phosphortrichlorid tropfenweise versetzt. Nach 8stuendigem Ruehren bei 80° C gibt man 43 ml 6 N Salzsaeure dazu, erhoeht die Temperatur auf 100° und ruehrt weitere 8 Stunden. Die gelbliche Suspension wird dann heiß filtriert, das Filtrat eingedampft und der Rueckstand, ein farbloses Oel, in 2 l Aceton eingeruehrt. Die Ausfaellung wird abgesaugt und mit Aceton und Ether gewaschen. Man erhaelt 4,6 g = 43 %, die in wenig Wasser geloest werden. Die Reinigung erfolgt durch Fraktionieren an der Amberlite-IR-120 $H^+$-Saeule. Die einzelnen Fraktionen werden elektrophoretisch auf Reinheit geprueft. Nach Vereinigen der reinen Fraktionen engt man die waessrige Loesung wieder ein und faellt erneut mit Aceton. Ausbeute = 2,9 g = 27 % weiße Kristalle, Fp. 143° C sintern, 155 - 158° C Aufschaeumen, Mrel: 0,29.

Beispiel 17

trans-3-(2-Aminocyclohexyl)-1-hydroxypropan-1,1-diphosphonsaeure

trans-3-(2-Aminocyclohexyl)propionsaeure-hydrochlorid wird nach C. A. 50, 9410 i mit 62 % Ausbeute hergestellt. Die Umsetzung mit phosphoriger Saeure/Phosphortrichlorid erfolgt wie im vorstehenden Beispiel beschrieben, ebenso die Aufarbeitung und Reinigung. Ausbeute: 10 %, Fp. 90°C sintern, bei 170° C Aufschaeumen, bei 220° C Zersetzung, Mrel: 0,27.

Beispiel 18

3-(2-Dimethylaminocyclohexyl)-1-hydroxypropan-1,1-diphosphonsaeure

3-(2-Dimethylaminocyclohexyl)propionsaeure wird wie in Beispiel 16 beschrieben mit phosphoriger Saeure/Phosphortrichlorid umgesetzt, hydrolysiert und auf reines Produkt aufgearbeitet. Es werden 40 % eines Dihydrates erhalten, das sich ab 65°C zersetzt, Mrel: o,27.
Das Ausgangsmaterial wird wie folgt erhalten:
3-(2-Aminophenyl)propionsaeure, aus o-Nitro-zimtsaurem Natrium durch Hydrierung an Pd-Kohle erhalten, wird mit Formalin in Gegenwart von Triethylamin weiter hydriert. Das entstandene Gemisch wird an der Kieselgelsaeule getrennt (Elutionsmittel: Methylenchlorid-Methanol-Essigsaeure = 9 : 1 : 0,2). Die gewünschte Verbindung, 3-(2-Dimethylaminophenyl)propionsaeure, wird mit 30 % als Oel erhalten. Die Kernhydrierung wird in Eisessig-Salzsaeure mit Pt-Katalysator durchgefuehrt und gibt das gewuenschte Ausgangsmaterial mit 44 %, Fp. 118°C sintern, 128 - 138°C.

Beispiel 19

3-(2-Amino-3-methylcyclohexyl)-1-hydroxypropan-1,1-diphosphonsaeure

3-Methyl-2-nitrobenzaldehyd, nach B. 47, 406 hergestellt, wird mit Malonsaeure in Pyridin und Piperidinzusatz zur 3-Methyl-2-nitrozimtsaeure umgesetzt.
Ausbeute: 64 % aus Methanol, Fp. 230 -235° C.
Nach Hydrierung in Natronlauge mit Pd-Katalysator werden 2 Mol Acetanhydrid zugesetzt und das Reaktionsprodukt an der Amberlite-IR 120 $H^+$-Saeule gereinigt. Man erhaelt so 75 % 3-(2-Acetylamino-3-methylphenyl)propionsaeure, Fp. 110° sintern, 117 - 120° C, die mit phosphoriger Saeure/Phosphortrichlorid analog Beispiel 16 umgesetzt wird. Nach der dort beschriebenen Aufarbeitungsmethode werden 9 % 3-(2-Amino-3-methylphenyl)-1-hydroxypropan-1,1-diphosphonsaeure vom Fp. 208° C sintern, 215 - 219° C Zersetzung erhalten. Die Kernhydrierung wird in waessriger Loesung mit Pt-Katalysator durchgefuehrt und liefert nach Reinigung an der Amberlite-IR-120 $H^+$-Saeule 67 % der gewuenschten Verbindung vom Fp. 133°C.sintern, 166°C Aufschaeumen, Mrel: 0.31.

Beispiel 20

3-(2-Aminocyclopentyl)-1-hydroxypropan-1,1-diphosphonsaeure

Ausgehend von 3-(2-Aminocyclopentyl)propionsaeure-hydrochlorid (beschrieben in C.A. 60, 4111 a) erhaelt man in analoger Weise wie in Beispiel 16 beschrieben die gewuenschte Verbindung mit 12 % Ausbeute als cis-trans-Gemisch (63:37), Fp. 105 - 125°C Zers., Mrel: 0.21.

Beispiel 21

3-(2-Aminocyclohexyl)-1-hydroxy-propan-1,1-diphosphonsäure

Ausgehend von 3-(2-Acetylaminocyclohexyl)-1-hydroxypropan-1,1-diphosphonsäure wird die Acetylgruppe vollständig durch 70 stündiges Erhitzen auf 100° C in 6 N Salzsäure eliminiert. Man reinigt die Diphosphonsäure durch Chromatographieren an Amberlite IR 120 $H^+$ Form und erhält so die 3-(2-Aminocyclohexyl)-1-hydroxypropan-1,1-diphosphonsäure x 1.5 Wasser als cis-trans-Gemisch (Verhältnis wie beim Ausgangsmaterial). Ausbeute: 23 %, Fp. 163° C aufschäumen, $M_{rel}$: 0.31.

**Patentansprüche**

1. Diphosphonate der allgemeinen Formel I

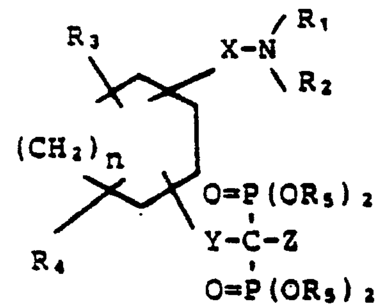

(I) ,

in der

$R_1$, $R_2$ jeweils unabhängig voneinander oder auch gemeinsam Wasserstoff, Acetyl oder $C_1$–$C_4$ Alkyl, das durch Phenyl, Pyridyl, Imidazolyl oder Thienyl substituiert sein kann,

$R_3$, $R_4$ jeweils unabhängig voneinander oder gemeinsam Wasserstoff, $C_1$–$C_4$ Alkyl oder zusammen $C_1$–$C_4$ Alkylen,

$R_5$ Wasserstoff oder $C_1$–$C_4$ Alkyl,

X einen Valenzstrich oder geradkettiges oder verzweigtes $C_1$–$C_4$ Alkylen,

Y einen Valenzstrich, geradkettiges oder verzweigtes, gegebenenfalls durch

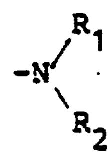

substituiertes $C_1$–$C_4$ Alkylen,

Z Wasserstoff, Hydroxy oder gegebenenfalls durch $C_1$–$C_4$ Alkylgruppen substituiertes Amino und

n = 1–3 bedeuten,

sowie deren pharmakologisch unbedenkliche Salze.

2. Diphosphonate der allgemeinen Formel I gemäß Anspruch 1, in der

$R_1$, $R_2$ jeweils unabhängig voneinander oder auch gemeinsam Wasserstoff, Methyl oder Acetyl sein können,

$R_3$, $R_4$ jeweils unabhängig voneinander Wasserstoff oder Methyl

$R_5$ Wasserstoff,

X, Y jeweils unabhängig voneinander einen Valenzstrich oder Methylen oder Ethylen,

Z Hydroxy und

n die Zahlen 1 und 2

bedeuten, sowie deren pharmakologisch unbedenkliche Salze.

3. Verfahren zur Herstellung von Diphosphonaten der allgemeinen Formel I

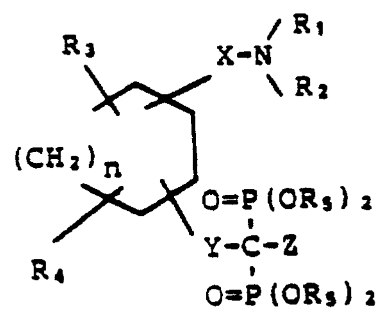

(I) ,

in der

$R_1$, $R_2$ jeweils unabhängig voneinander oder auch gemeinsam Wasserstoff, Acetyl oder $C_1$–$C_4$ Alkyl, das durch Phenyl, Pyridyl, Imidazolyl oder Thienyl substituiert sein kann,

$R_3$, $R_4$ jeweils unabhängig voneinander oder gemeinsam Wasserstoff, $C_1$–$C_4$ Alkyl oder zusammen $C_1$–$C_4$ Alkylen,

$R_5$ Wasserstoff oder $C_1$–$C_4$ Alkyl,

X einen Valenzstrich oder geradkettiges oder verzweigtes $C_1$–$C_4$ Alkylen,

Y einen Valenzstrich, geradkettiges oder verzweigtes, gegebenenfalls durch

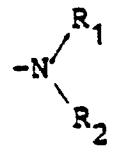

substituiertes $C_1$–$C_4$-Alkylen,
Z Wasserstoff, Hydroxy oder gegebenenfalls durch $C_1$–$C_4$ Alkylgruppen substituiertes Amino und
n = 1–3 bedeuten,
sowie deren pharmakologisch unbedenkliche Salze, dadurch gekennzeichnet, daß man
I. für den Fall, daß Z in der allgemeinen Formel I OH bedeutet,
a) eine Carbonsäure der allgemeinen Formel II

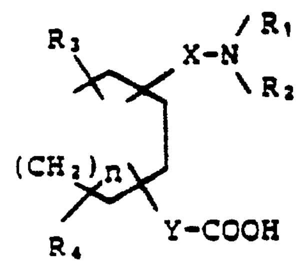

(II),

in der $R_1$–$R_4$, X, Y und n die oben angegebenen Bedeutungen haben, mit einem Gemisch aus phosphoriger Säure bzw. Phosphorsäure und einem Phosphorhalogenid umsetzt und anschließend zur freien Diphosphonsäure verseift,
oder
b) ein Carbonsäurechlorid der allgemeinen Formel III

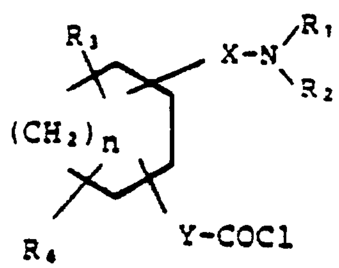

(III),

in der $R_1$, $R_2$, $R_3$, $R_4$, X, Y und n die oben angegebenen Bedeutungen haben, und falls

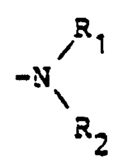

eine primäre oder sekundäre Aminogruppe darstellt, diese Gruppe geschützt ist, wobei $R_1$ und $R_2$ zusammen auch den Phthaloylrest bedeuten können, mit einem Trialkylphosphit der allgemeinen Formel IV

$P(OR')_3$ (IV),

in der R' niederes Alkyl bedeutet, zu einem Acylphosphonat der allgemeinen Formel V

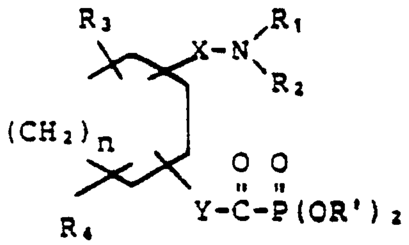

(V),

in der $R_1$–$R_4$, X, Y, n und R' die oben angegebenen Bedeutungen haben, umsetzt, anschließend mit einem Dialkylphosphit der allgemeinen Formel VI

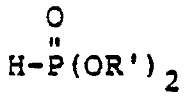

(VI),

in der R' die oben angegebene Bedeutung hat, zu einem Diphosphonat der allgemeinen Formel VII

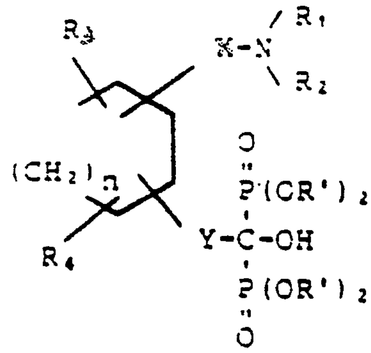

(VII),

in der $R_1$–$R_4$, X, Y, n und R' die oben angegebenen Bedeutungen haben, reagieren läßt, gegebenenfalls vorhandene Schutzgruppen abspaltet und gegebenenfalls die entstandenen Tetraester zu Diestern oder Säuren der allgemeinen Formel I verseift,
oder
c) eine Verbindung der allgemeinen Formel I'

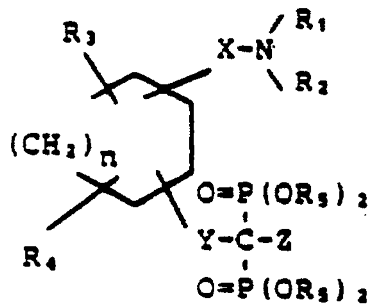

(I'),

in der $R_3$, $R_4$, $R_5$, n, X und Y die oben angegebenen Bedeutungen haben, $R_1$ und $R_2$ jeweils niederes Acyl oder Alkyl, das durch Aryl substituiert sein kann oder zusammen einen Phthaloylrest bedeuten und $Z = NH_2$ darstellt,
diazotiert,
oder
II. für den Fall, daß Z in der allgemeinen Formel I gegebenenfalls durch Alkylgruppen substituiertes Amino bedeutet,
ein Carbonsäurederivat der allgemeinen Formel VIII

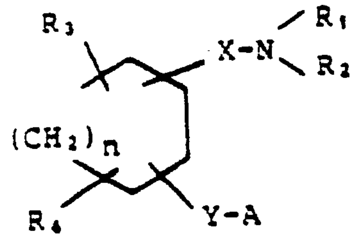

(VIII),

in der $R_1$–$R_4$, X, Y und n die oben angegebenen Bedeutungen haben und A eine Nitril-, Iminoether- oder eine gegebenenfalls am Stickstoff durch niederes Alkyl substituierte Carboxamidogruppe darstellt,
mit einer Phosphorverbindung der allgemeinen Formel IX

$$PT_3 \qquad (IX),$$

in der T = Halogen, OH oder OR' bedeutet, wobei R' die oben angegebene Bedeutung hat, umsetzt und gegebenenfalls anschließend verseift
oder
III. für den Fall, daß Z in der allgemeinen Formel I Wasserstoff bedeutet,
eine Verbindung der allgemeinen Formel X

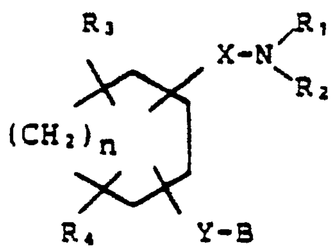

(X),

in der $R_1$–$R_4$, X, Y und n die oben angegebenen Bedeutungen haben und falls

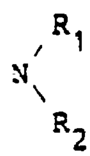

eine primäre oder sekundäre Aminogruppe darstellt, diese Gruppe geschützt ist, wobei $R_1$ und $R_2$ zusammen auch den Phthaloylrest bedeuten können, und B einen reaktiven Rest darstellt, mit einer Verbindung der allgemeinen Formel XI

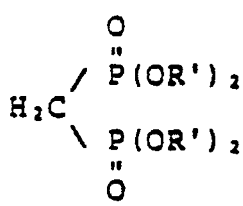

(XI),

in der R' die oben angegebene Bedeutung hat, zu einem Diphosphonat der allgemeinen Formel XII

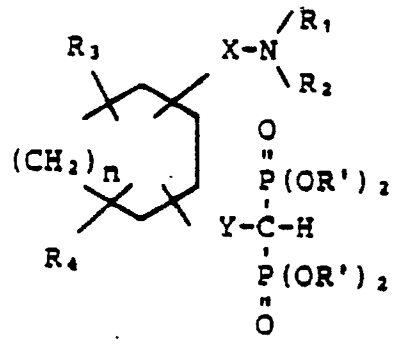

(XII),

in der $R_1$–$R_4$, X, Y, n und R' die oben angegebenen Bedeutungen haben, umsetzt, gegebenenfalls vorhandene Schutzgruppen abspaltet und gegebenenfalls die entstandenen Tetraester zu Diestern oder Säuren der allgemeinen Formel I verseift und gewünschtenfalls vorhandene N-Acyl-Substituenten abspaltet und anschließend gewünschtenfalls freie Aminogruppen alkyliert oder acyliert und die erhaltenen Verbindungen der Formel I in pharmakologisch verträgliche Salze überführt.

4. Verfahren zur Herstellung von Diphosphonaten der allgemeinen Formel I gemäß Anspruch 3, in der $R_1$, $R_2$ jeweils unabhängig voneinander oder auch gemeinsam Wasserstoff, Methyl oder Acetyl sein können,
$R_3$, $R_4$ jeweils unabhängig voneinander Wasserstoff oder Methyl
$R_5$ Wasserstoff,
X, Y jeweils unabhängig voneinander einen Valenzstrich oder Methylen oder Ethylen,
Z Hydroxy und
n die Zahlen 1 und 2
bedeuten, sowie deren pharmakologisch unbedenkliche Salze.

5. Arzneimittel, enthaltend mindestens eine Verbindung gemäß Anspruch 1 oder 2, sowie ggf. übliche Träger- und/oder Hilfsstoffe.

6. Verwendung von Verbindungen gemäß Anspruch 1 oder 2 zur Herstellung von Arzneimitteln zur Behandlung von Calciumstoffwechselstörungen.

## Claims

1. Diphosphonates of the general formula I

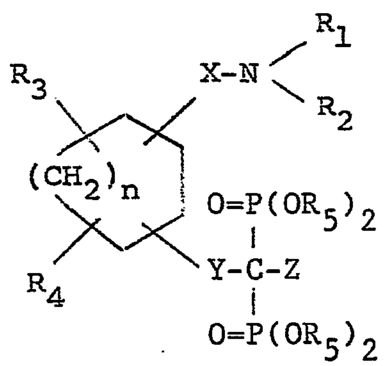

(I)

in which $R_1$, $R_2$, in each case independently of one another or also together signify hydrogen, acetyl or $C_1$–$C_4$-alkyl which can be substituted by phenyl, pyridyl, imidazolyl or thienyl, $R_3$, $R_4$ in each case independently of one another or together signify hydrogen, $C_1$–$C_4$-alkyl or together $C_1$–$C_4$-alkylene, $R_5$ hydrogen or $C_1$–$C_4$-alkyl, X a valency bond or straight-chained or branched $C_1$–$C_4$-alkylene, Y a valency bond, straight-chained or branched $C_1$–$C_4$-alkylene possibly substituted by

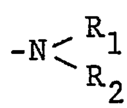

,

Z hydrogen, hydroxyl or amino group possibly substituted by $C_1$–$C_4$-alkyl groups and $\underline{n}$ = 1–3, as well as their pharmacologically acceptable salts.

2. Diphosphonates of the general formula I according to claim 1, in which $R_1$, $R_2$, in each case independently of one another or also together can be hydrogen, methyl or acetyl radicals, $R_3$, $R_4$ in each case independently of one another signify hydrogen or methyl, $R_5$ hydrogen atom, X, Y in each case independently of one another a valency bond or methylene or ethylene, Z hydroxyl and n the numbers 1 and 2, as well as their pharmacologically acceptable salts.

3. Process for the preparation of diphosphonates of the general formula I

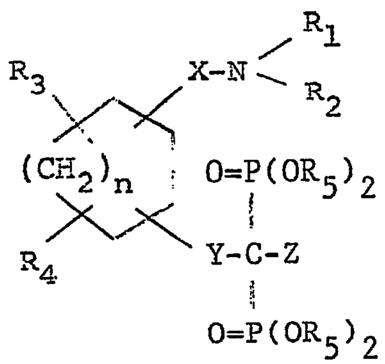

(I)

in which $R_1$, $R_2$, in each case independently of one another or also together can be hydrogen, acetyl or $C_1$–$C_4$-alkyl which can be substituted by phenyl, pyridyl, imidazolyl or thienyl, $R_3$, $R_4$ in each case independently of one another or together signify hydrogen, $C_1$–$C_4$-alkyl or together $C_1$–$C_4$-alkylene, $R_5$ hydrogen or $C_1$–$C_4$-alkyl, X a valency bond or straight-chained or branched $C_1$–$C_4$-alkylene, Y a valency bond, straight-chained or branched $C_1$–$C_4$-alkylene possibly substituted by

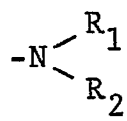

,

Z hydrogen, hydroxyl group or amino possibly substituted by $C_1$–$C_4$-alkyl groups and n = 1–3, as well as of their pharmacologically acceptable salts, characterised in that one

I. for the case that Z in the general formula I signifies OH,

a) reacts a carboxylic acid of the general formula II

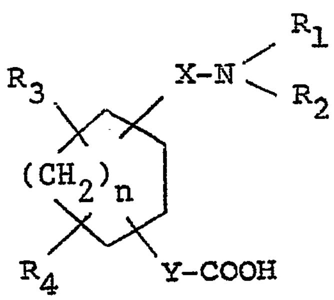

(II)

in which $R_1$–$R_4$, X, Y and n have the above-given meanings, with a mixture of phosphorus acid or phosphoric acid and a phosphorus halide and subsequently saponifies to the free diphosphonic acid; or

b) reacts a carboxylic acid chloride of the general formula III

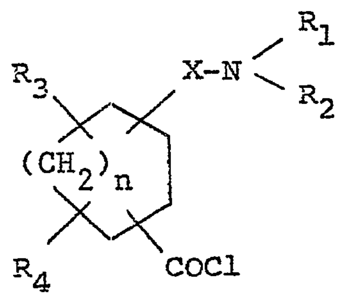

(III)

in which $R_1$, $R_2$, $R_3$, $R_4$, X, Y and n have the above-given meanings and, if

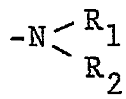

represents a primary or secondary amino group, this group is protected, whereby $R_1$ and $R_2$ can together also signify a phthaloyl radical, with a trialkyl phosphite of the general formula IV

$P(OR')_3$ (IV)

in which R' signifies lower alkyl, to an acyl phosphonate of the general formula V

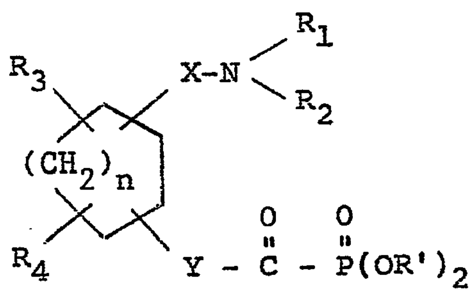

(V)

in which $R_1$–$R_4$, X, Y, n and R' have the above-given meanings, subsequently allows to react with a dialkyl phosphite of the general formula VI

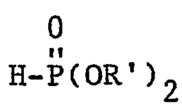

(VI)

in which R' has the above-given meaning, to a diphosphonate of the general formula VII

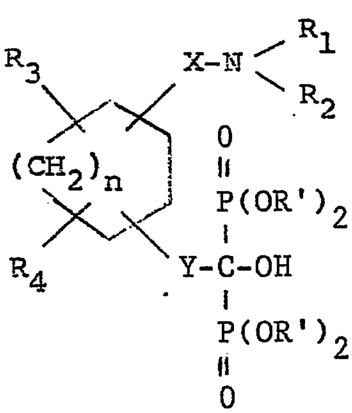

(VII)

in which $R_1$–$R_4$, X, Y, n and R' have the above-given meanings, splits off protective groups possibly present and possibly saponifies the resultant tetraesters to diesters or acids of the general formula I, or

c) diazotises a compound of the general formula I'

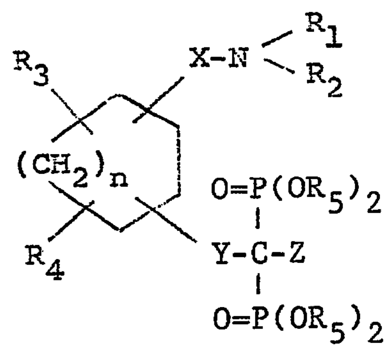

(I')

in which $R_3$, $R_4$, $R_5$, n, X and Y have the above-given meanings, $R_1$ and $R_2$ each represent lower acyl or alkyl which can be substituted by aryl or together signify a phthaloyl radical and $Z = NH_2$; or

II. for the case that Z in the general formula I signifies an amino possibly substituted by alkyl groups, reacts a carboxylic acid derivative of the general formula VIII

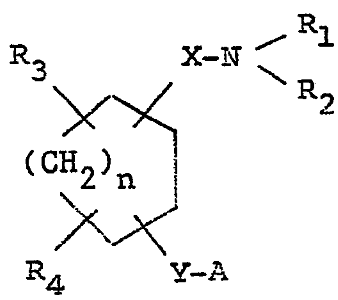

(VIII)

in which $R_1$–$R_4$, X, Y and n have the above-given meanings and A represents a nitrile, imino ether or a carboxamide group possibly substituted on the nitrogen atom by lower alkyl, with a phosphorus compound of the general formula IX

$$PT_3 \qquad (IX)$$

in which T = halogen atom, OH or OR', whereby R' has the above-given meaning, and possibly subsequently saponifies; or

III. for the case that Z in the general formula I signifies hydrogen, reacts a compound of the general formula X

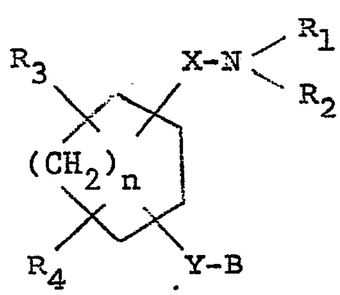

(X)

in which $R_1$–$R_4$, X, Y and n have the above-given meanings and if

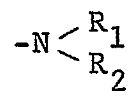

represents a primary or secondary amino group, this is protected, whereby $R_1$ and $R_2$ together can also represent the phthaloyl radical and B represents a reactive residue, with a compound of the general formula XI

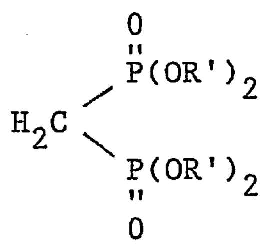

(XI)

in which R' has the above-given meaning, to a diphosphonate of the general formula XII

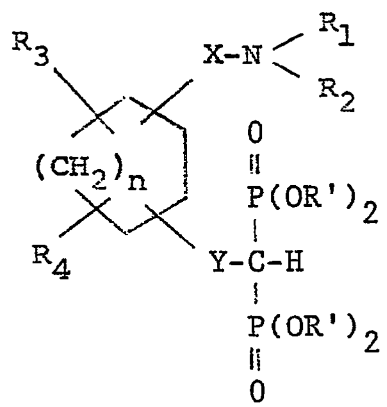

(XII)

in which $R_1$–$R_4$, X, Y, n and R' have the above-given meanings, splits off protective groups possibly present and possibly saponifies the resultant tetraesters to diesters or acids of the general formula I; and, if desired, splits off N-acyl substituents present and subsequently, if desired, alkylates or acylates free amino groups and converts the compounds obtained of the formula I into pharmacologically acceptable salts.

4. Process for the preparation of diphosphonates of the general formula I according to claim 3, in which $R_1$, $R_2$ in each case independently of one another or also together can be hydrogen, methyl or acetyl, $R_3$, $R_4$ in each case independently of one another hydrogen or methyl, $R_5$ hydrogen atom, X, Y in each case independently of one another a valency bond or methylene or ethylene, Z hydroxyl and n the numbers 1 and 2, as well as of their pharmacologically acceptable salts.

5. Medicaments containing at least one compound according to claim 1 or 2, as well as possibly usual carrier and/or adjuvant materials.

6. Use of compounds according to claim 1 or 2 for the preparation of medicaments for the treatment of calcium metabolism disturbances.

**Revendications**

1. Diphosphonates de formule générale I

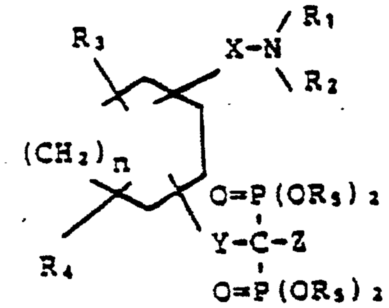

(I),

où $R_1$, $R_2$ représentent, chacun indépendamment ou même conjointement, un atome d'hydrogène, un groupe acétyle ou un groupe alkyle en $C_1$–$C_4$ qui peut être substitué par un groupe phényle, pyridyle, imidazolyle ou thiényle,

$R_3$, $R_4$ représentent, chacun indépendamment ou conjointement, un atome d'hydrogène, un groupe alkyle en $C_1$–$C_4$, ou en commun un groupe alkylène en $C_1$–$C_4$,

$R_5$ représente un atome d'hydrogène ou un groupe alkyle en $C_1$–$C_4$,

X représente une valence libre ou un groupe alkylène en $C_1$–$C_4$, à chaîne droite ou ramifiée,

Y représente une valence libre ou un groupe alkylène en $C_1$–$C_4$, à chaîne droite ou ramifiée, éventuellement par un groupe

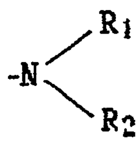

Z représente un atome d'hydrogène, un groupe hydroxyle ou un groupe amino éventuellement substitué par un groupe alkyle en $C_1$–$C_4$, et

n = 1–3,

ainsi que leurs sels physiologiquement acceptables.

2. Diphosphonates de formule générale I, selon la revendication 1, où

$R_1$, $R_2$ représentent, chacun indépendamment ou même conjointement, un atome d'hydrogène, un groupe méthyle ou acétyle,

$R_3$, $R_4$ représentent, chacun indépendamment, un atome d'hydrogène ou un groupe méthyle,

$R_5$ représente un atome d'hydrogène,

X, Y représentent, chacun indépendamment, une valence libre ou un groupe méthylène ou éthylène,

Z représente un groupe hydroxyle et

n représente 1 et 2,

ainsi que leurs sels physiologiquement acceptables.

3. Procédé de fabrication de diphosphonates de formule générale I,

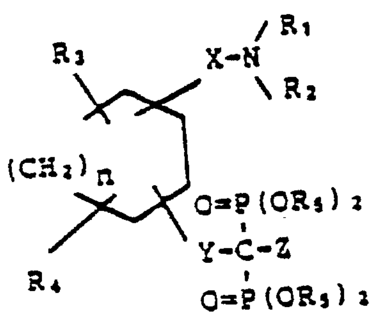

(I),

dans laquelle

$R_1$, $R_2$ représentent, chacun indépendamment ou même conjointement, un atome d'hydrogène, un groupe acétyle ou un groupe alkyle en $C_1$–$C_4$, qui peut être substitué par un groupe phényle, pyridyle, imidazolyle ou thiényle,

$R_3$, $R_4$ représentent, chacun indépendamment ou conjointement, un atome d'hydrogène, un groupe alkyle en $C_1$–$C_4$, ou en commun un groupe alkylène en $C_1$–$C_4$,

$R_5$ représente un atome d'hydrogène ou un groupe alkyle en $C_1$–$C_4$,
X représente une valence libre ou un groupe alkylène en $C_1$–$C_4$, à chaîne droite ou ramifiée,
Y représente une valence libre ou un groupe alkylène en $C_1$–$C_4$, à chaîne droite ou ramifiée, éventuellement substitué par un groupe

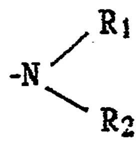

Z représente un atome d'hydrogène, un groupe hydroxyle ou un groupe amino éventuellement substitué par un groupe alkyle en $C_1$–$C_4$, et
n = 1–3,
ainsi que leurs sels physiologiquement acceptables, caractérisé en ce que
I. dans le cas où Z représente, dans la formule générale I, un groupe OH,
a) on fait réagir un acide carboxylique de formule générale II,

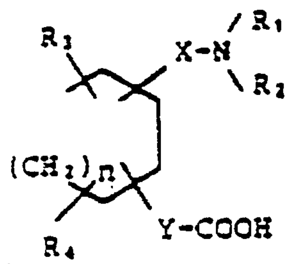 (II),

dans laquelle $R_1$–$R_4$, X, Y et n ont les significations mentionnées ci-dessus, avec un mélange d'acide phosphorique ou phosphoreux et d'un halogénure de phosphore, et ensuite, on saponifie en acide diphosphonique libre,
ou
b) on fait réagir un chlorure d'acide carboxylique de formule III

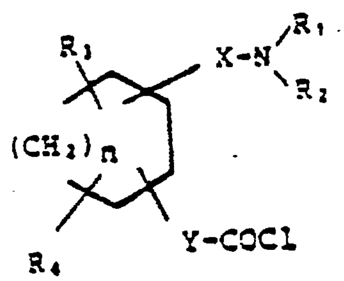 (III),

où $R_1$, $R_2$, $R_3$, $R_4$, X, Y et n ont les significations mentionnées ci-dessus, et dans le cas où

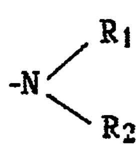

représente un groupe amino primaire ou secondaire, ce groupe étant protégé, $R_1$ et $R_2$ pouvant représenter en commun également le groupe phtaloyle, avec un phosphite de trialkyle de formule générale IV

$$P(OR')_3 \qquad (IV),$$

ou R' représente un groupe alkyle inférieur, pour obtenir un phosphonate d'acyle de formule générale V

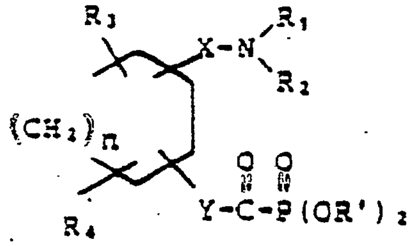 (V),

où $R_1$–$R_4$, X, Y, n et R' ont les significations mentionnées ci-dessus, ensuite on laisse réagir avec un phosphite de dialkyle de formule générale VI

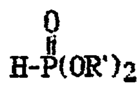 (VI),

où R' a la signification mentionnée plus haut, en un diphosphonate de formule générale VII

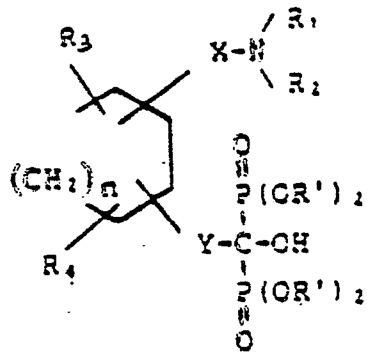 (VII),

où $R_1$–$R_4$, X, Y, n et R' ont les significations mentionnées ci-dessus, on libère les groupes de protection éventuels et on saponifie éventuellement le tétraester produit en diester ou en acide de formule générale I,

ou

c) on diazote un composé de formule générale I'

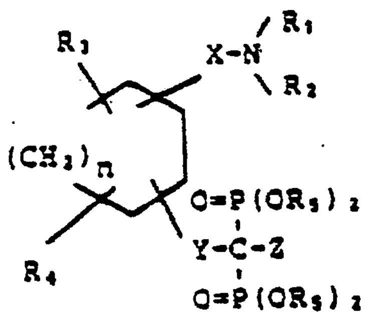 (I'),

où $R_3$, $R_4$, $R_5$, n, X et Y ont les significations mentionnées ci-dessus, $R_1$ et $R_2$ représentent chacun un groupe acyle ou alkyle inférieur qui peut être substitué par un groupe aryle, ou conjointement, un groupe phtaloyle, et $Z = NH_2$,

ou

II. dans le cas où Z représente, dans la formule générale I, un groupe amino éventuellement substitué par un groupe alkyle,

on fait réagir un dérivé d'acide carboxylique de formule générale VIII

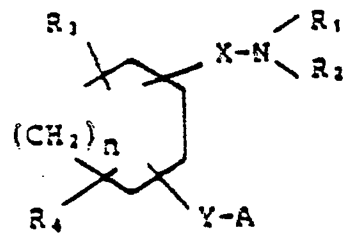

(VIII),

où $R_1$–$R_4$, X, Y et n ont les significations mentionnées ci-dessus et A représente un groupe nitrile, iminoéther ou un groupe carboxyamido éventuellement substitué sur l'azote par un groupe alkyle inférieur,
avec un composé du phosphore de formule générale IX,

$$PT_3 \qquad (IX),$$

dans lequel T = un atome d'halogène, un groupe OH ou OR', R' ayant la signification mentionnée ci-dessus, et ensuite, on le saponifie éventuellement,
ou
III. dans le cas où Z représente, dans la formule générale I, un atome d'hydrogène,
on fait réagir un composé de formule générale X

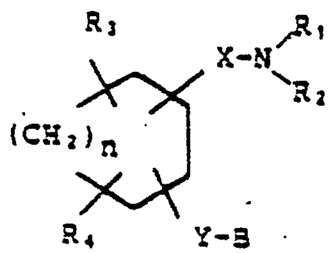

(X),

où $R_1$–$R_4$, X, Y et n ont les significations mentionnées ci-dessus, et dans le cas où

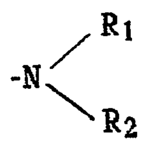

représente un groupe amino primaire ou secondaire, ce groupe étant protégé, $R_1$ et $R_2$ pouvant représenter en commun également le groupe phtaloyle, et B représentant un groupe réactif, avec un composé de formule générale XI

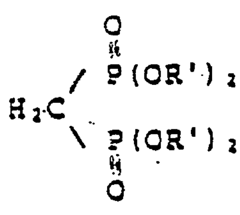

(XI),

où R' a la signification mentionnée ci-dessus, pour obtenir un diphosphonate de formule générale XII

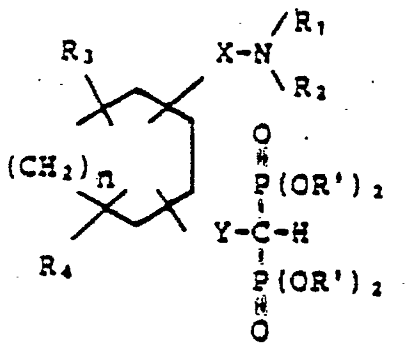

(XII),

où $R_1$–$R_4$, X, Y, n et R' ont les significations mentionnées ci-dessus, on libère les groupes de protection éventuels et on saponifie éventuellement le tétraester produit en diester ou acide de formule générale I,

et on libère éventuellement les substituants N-acyle, et ensuite, on alkyle ou acyle éventuellement les groupes amino libres et on transforme les composés de formule générale I obtenus en leurs sels physiologiquement acceptables.

4. Procédé de fabrication des diphosphonates de formule générale I selon la revendication 3, où

$R_1$, $R_2$ représentent, chacun indépendamment ou même conjointement, un atome d'hydrogène, un groupe méthyle ou acétyle,

$R_3$, $R_4$ représentent, chacun indépendamment, un atome d'hydrogène ou un groupe méthyle,

$R_5$ représente un atome d'hydrogène,

X, Y représentent, chacun indépendamment, une valence libre ou un groupe méthylène ou éthylène,

Z représente un groupe hydroxyle et

n représente 1 et 2,

ainsi que leurs sels physiologiquement acceptables.

5. Produit pharmaceutique contenant au moins un composé selon la revendication 1 ou 2, ainsi qu'éventuellement des supports et/ou des additifs usuels.

6. Utilisation des composés selon la revendication 1 ou 2, pour la fabrication de produits pharmaceutiques destinés au traitement des troubles du métabolisme de calcium.